# EUROPÄISCHE PATENTSCHRIFT

(11) **EP 0 979 649 B1**
(45) Veröffentlichungstag und Bekanntmachung des Hinweises auf die Patenterteilung: **09.07.2003**
(21) Anmeldenummer: 99115819.7
(22) Anmeldetag: 11.08.1999
(51) Int. Cl.: A61K 9/20, A61K 9/22, A61K 47/32, A61K 31/138

(54) **Verwendung von N-Vinylpyrrolidon-und Vinylacetat-haltigen Copolymeren als Matrix zur Herstellung von festen, oralen, pharmazeutischen und kosmetischen Zubereitungen**
Use of copolymers comprising N-vinylpyrrolidone and vinylacetate for the production of solid oral pharmaceutic and cosmetic compositions
Utilisation de copolymères comprenant de la N-vinylpyrrolidone et du vinylacetate pour la production de formes solides orales pharmaceutiques et cosmétiques

(30) Priorität: 13.08.1998 DE 19836646
(43) Veröffentlichungstag der Anmeldung: 16.02.2000
(73) Patentinhaber: BASF AKTIENGESELLSCHAFT, 67056 Ludwigshafen (DE)
(72) Erfinder: Meffert, Helmut, Dr., 68161 Mannheim (DE); Ruchatz, Folker, Dr., 67433 Neustadt (DE)

(56) Entgegenhaltungen:
- EP-A- 0 545 209
- DE-A- 3 810 343
- US-A- 3 851 032
- US-A- 4 551 512
- US-A- 4 647 599

## Beschreibung

Die vorliegende Erfindung betrifft die Verwendung von N-Vinylpyrrolidon- und Vinylacetat-haltigen Copolymeren als Matrix zur Herstellung von festen, oralen pharmazeutischen und kosmetischen Zubereitungen.

Orale Arzneiformen mit einer verzögerten Wirkstofffreigabe (Retardarzneiformen) gewinnen zunehmend an Bedeutung. Mit ihr sind vorteilhafterweise eine verbesserte Patientencompliance durch eine reduzierte Einnahmefrequenz, eine Verringerung von Nebenwirkungen durch Vermeidung von Plasmaspiegelspitzen, gleichmäßigere Blutspiegel des Arzneistoffs sowie die Vermeidung von lokalen Irritationen verbunden.

Neben Coating-Retardformen, d.h. Formulierung von arzneistoffhaltigen Kernen, die mit einem wasserunlöslichen aber semipermeablen bzw. porenhaltigen Film überzogen werden, durch die der Arzneistoff diffundiert, kann die Steuerung und Verlängerung der Freisetzung auch durch eine Einbettung des Arzneistoffes in einer Matrix erreicht werden.

Besonders die Einbettung des Arzneistoffs in eine Matrix bietet die Vorteile einer einfachen und preiswerten Herstellung und einer hohen Arzneimittelsicherheit, da Dose Dumping Effekte (z.B. das Auftreten hoher Plasmakonzentrationen durch falsche Einnahme - beispielsweise das Zerkauen statt Schlucken von gecoateten Tabletten) nicht auftreten können.

Die hierfür in der Regel eingesetzten Hilfsstoffe wie Hydroxypropylcellulose, Hydroxypropylmethylcellulose, Alginsäure bzw. Alginate sowie Xanthan besitzen jedoch anwendungstechnische Nachteile. Diese liegen zum einen in einer pH-Wert- bzw. Ionenstärkeabhängigen Arzneistofffreigabe und zum anderen in einer unbefriedigenden Direkttablettierbarkeit, da aufgrund der geringen Bindemittelwirkung und der schlechten Fließeigenschaften der oben genannten Polymere die resultierenden Preßlinge häufig nur eine geringe Härte aufweisen bzw. inhomogen sind.

Da es sich bei den oben genannten Hilfsstoffen teilweise um Produkte natürlichen Ursprungs bzw. um weiterveredelte Produkte natürlichen Ursprungs handelt, kann es zu Schwankungen in der Chargenkonformität und somit zu einer ungünstigen Beeinflussung der Performance der pharmazeutischen Zubereitung kommen.

DE-A-38 10 343 beschreibt ein Verfahren zur Herstellung von festen pharmazeutischen Retardformen, die als Bindemittel ein N-Vinylpyrrolidon/Vinylacetat-haltiges wasserlösliches Copolymer mit einem K-Wert von 15 bis 35 enthalten, wobei man den pharmazeutischen Wirkstoff, das polymere Bindemittel und gegebenenfalls weitere galenische Hilfsstoffe unterhalb der Glastemperatur des Bindemittels mischt, diese Mischung oberhalb der Glastemperatur des Bindemittels, aber unterhalb der Zersetzungstemperatur des Wirkstoffs zu pharmazeutischen Formen verpreßt und bei einer Temperatur der Form unterhalb der Glastemperatur des Bindemittels entformt.

WO 97/11688 beschreibt die Verwendung von N-Vinylpyrrolidon/Vinylacetat-haltigen Copolymeren als Matrix für die transdermale Applikation von Wirkstoffen.

EP545209 beschreibt ein redispergierbares, rieselfähiges Dispersionspulver aus einem 15 bis 40 Gew.-% Vinylpyrrolidon einpolymerisiert enthaltenden Vinylpyrrolidon-Vinylacetat-Copolymeren.

US4647599 offenbart wirkstofffreie matrixbildende Granalien, geeignet zur Herstellung von pharmazeutischen Präparaten, insbesondere Tabletten, mit geregelter beziehungsweise verzögerter Wirkstoffabgabe.

US4551512 offenbart ein leicht wasserlösliches und schwach hygroskopisches Terpolymer aus Vinylpyrrolidon, Vinylacetat oder Vinylpropionat und Hydroxyethylacrylat, Hydroxypropylacrylat oder Hydroxyethylmethacrylat. Die Anwendung als Hilfstoffe in der Kosmetik und Pharmazie wird ebenfalls beansprucht.

US3851032 offenbart ein Copolymer Matrix die einen crystallisierten Wirkstoff enhält.

Der vorliegenden Erfindung lag die Aufgabe zugrunde, Polymere zu finden, die als Matrix zur Herstellung von festen, oralen, pharmazeutischen und kosmetischen Zubereitungen mit kontrollierter Wirkstofffreigabe geeignet sind.

Diese Aufgabe wurde gelöst durch die Verwendung von Copolymeren mit einem K-Wert von 50 bis 200, enthaltend
a) 15 bis 50 Gew.-% N-Vinylpyrrolidon und
b) 85 bis 50 Gew.-% Vinylacetat,
wobei sich die Gew.-% Angaben der Einzelkomponenten zu 100 % addieren, als Matrix zur Herstellung von festen, oralen, pharmazeutischen und kosmetischen Zubereitungen.

Der Anteil an N-Vinylpyrrolidon im Copolymerisat liegt im Bereich von 15 bis 50 Gew.-%, bevorzugt 20 bis 40 Gew.-%, besonders bevorzugt im Bereich von 25 bis 35 Gew.-%.

Der Anteil an Vinylacetat im Copolymerisat liegt im Bereich von 85 bis 50 Gew.-%, bevorzugt 80 bis 60 Gew.-%, besonders bevorzugt im Bereich von 75 bis 65 Gew.-%.

Gegebenenfalls kann es sinnvoll sein, neben den bereits genannten Monomerbausteinen a) und b) die im folgenden aufgezählten Comonomeren c) für die Polymerisation zu verwenden:

Monoethylenisch ungesättigte Carbonsäuren mit 3 bis 8 C-Atomen wie Acrylsäure, Methacrylsäure, Dimethacrylsäure, Ethacrylsäure, Maleinsäure, Citraconsäure, Methylenmalonsäure, Allylessigsäure, Vinylessigsäure Crotonsäure, Fumarsäure, Mesaconsäure und Itaconsäure. Aus dieser Gruppe von Monomeren verwendet man vorzugsweise Acrylsäure, Methacrylsäure, Maleinsäure oder Mischungen der genannten Carbonsäuren.

Die monoethylenisch ungesättigten Carbonsäuren können in Form der freien Säure und - soweit vorhanden - der Anhydride oder in partiell oder in vollständig neutralisierter Form bei der Copolymerisation eingesetzt werden. Zur Neutralisation verwendet man vorzugsweise Alkalimetall- oder Erdalkalimetallbasen, Ammoniak oder Amine, z.B. Natronlauge, Kalilauge, Soda, Pottasche, Natriumhydrogencarbonat, Magnesiumoxid, Calciumhydroxid, Calciumoxid, gasförmiges oder wäßriges Ammoniak, Triethylamin, Ethanolamin, Diethanolamin, Triethanolamin, Morpholin, Diethylentriamin oder Tetra-ethylenpentamin.

Weitere geeignete Comonomere c) sind beispielsweise die Ester, Amide und Nitrile der oben angegebenen Carbonsäuren, z.B. Acrylsäuremethylester, Acrylsäureethylester, Methacrylsäure-methylester, Methacrylsäureethylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Acrylamid, Methacrylamid, N,N-Dimethylacrylamid, N-tert.-Butylacrylamid, Acrylnitril, Methacrylnitril, Dimethylaminoethylacrylat, Diethylaminoethylacrylat, Diethylaminoethylmethacrylat sowie die Salze der zuletzt genannten Monomeren mit Carbonsäuren oder Mineralsäuren sowie die guaternierten Produkte.

Außerdem eignen sich als andere copolymerisierbare Monomere Acrylamidoglycolsäure, Vinylsulfonsäure, Allylsulfonsäure, Methallylsulfonsäure, Styrolsulfonsäure, Acrylsäure-(3-sulfopropyl)ester, Methacrylsäure(3-sulfopropyl)ester und Acrylamidomethylpropansulfonsäure sowie Phosphonsäuregruppen enthaltende Monomere, wie Vinylphosphonsäure, Allylphosphonsäure und Acrylamidomethanpropanphosphonsäure.

Weitere geeignete copolymerisierbare Verbindungen sind N-Vinylimidazol, N-Vinyl-2-methylimidazol, N-Vinyl-4-methylimidazol, Diallylammoniumchlorid und Vinylpropionat. Es ist selbstverständlich auch möglich, Mischungen der genannten Monomeren einzusetzen.

Der Anteil der Monomerbausteine c) im Copolymerisat kann im Bereich von 0 bis 30 Gew.-%, bevorzugt 0 bis 20 Gew.-%, besonders bevorzugt im Bereich von 0 bis 10 Gew.-% liegen, wobei sich die Gew.-% Angaben der Komponenten a) bis c) zu 100 % addieren.

Die Herstellung der Copolymerisate erfolgt nach bekannten Verfahren, z.B. der Lösungs-, Fällungs-, Emulsions- oder umgekehrte Suspensionspolymerisation unter Verwendung von Verbindungen, die unter den Polymerisationsbedingungen Radikale bilden.

Die Polymerisationstemperaturen liegen üblicherweise in dem Bereich von 30 bis 200, vorzugsweise 40 bis 110°C.

Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, z.B. Natriumsulfit, Natriumbisulfit, Natriumformaldehydsulfoxilat und Hydrazin.

Die Copolymeren besitzen K-Werte von mindestens 50, vorzugsweise 50 bis 200, besonders bevorzugt 55 bis 150, ganz besonders bevorzugt 60 bis 100. Die K-Werte werden bestimmt nach H. Fikentscher, Cellulose-Chemie, Band 13, 58 bis 64 und 71 bis 74 (1932) in wäßriger oder alkoholischer Lösung bei 25°C, bei Konzentrationen, die je nach K-Wert-Bereich zwischen 0,1 % und 5 % liegen.

Zur Erhöhung der Molekulargewichte der erfindungsgemäß verwendeten Copolymere können zusätzlich Quervernetzer in Konzentrationen von bis zu 5 Gew.-%, bevorzugt 0,2 bis 3 Gew.-%, bezogen auf die übrigen Monomerbausteine, bei der Polymerisation zugesetzt werden.

Als Vernetzungsreagentien geeignet sind u.a. Divinylether von aliphatischen Diolen, beispielsweise Divinylether von 1,3-Butandiol, 1,4-Butandiol, 1,5-Pentandiol, 1,6-Hexandiol, 1,7-Heptandiol, 1,8-Octandiol, 1,9-Nonandiol 1,10-Decandiol, 1,11-Undecandiol und 1,12-Dodecandiol sowie Divinylether von Diethylenglykol, Triethylenglykol bis hin zu Polyethylenglykol mit einem Molekulargewicht von bis zu 6000.

Weitere Vernetzungsreagentien sind N,N'-Divinylimidazolidon, Divinylbenzol, Allyletherderivate von Polyhydroxyalkoholen, Triallylamine, Tetraallylethylendiamine sowie Diallylphthalate.

Bevorzugte copolymerisierbare Vernetzer sind N,N'-Divinylimidazolidon, Divinylethylenharnstoff, Pentaerythritol-triallylether.

Die erhaltenen Polymer-Dispersionen oder Lösungen können durch verschiedene Trocknungsverfahren wie z.B. Sprühtrocknung, Fluidized Spray Drying, Walzentrocknung oder Gefriertrocknung in Pulverform überführt werden.

Die Polymere eignen sich erfindungsgemäß als Matrix zur Herstellung von kosmetischen und pharmazeutischen Darreichungsformen, insbesondere von festen, oral anwendbaren Zubereitungen, bei denen der oder die Wirkstoffe verzögert freigegeben werden sollen.

Je nach Menge und Zusammensetzung und der daraus resultierenden Quellungseigenschaften der erfindungsgemäß verwendeten Polymere läßt sich dabei die Freisetzungsgeschwindigkeit der Wirkstoffe gezielt verändern.

Überraschenderweise zeigten die erfindungsgemäß verwendeten Copolymeren mit K-Werten größer 50 und einem Gehalt an Vinylacetat im Copolymer von 50 bis 85 Gew.-% deutlich bessere Retardierungseigenschaften als die bislang bekannten N-Vinylpyrrolidon/Vinylacetat Copolymeren, beispielsweise Kollidon® VA 64 (Fa. BASF, mit einem Gehalt an Vinylacetat von 40 Gew.-%).

Gegenstand der Erfindung sind daher auch feste, orale, pharmazeutische und kosmetische Zubereitungen, enthaltend als Matrix mindestens ein Copolymer mit einem K-Wert von 50 bis 200 aus
a) 15 bis 50 Gew.-% N-Vinylpyrrolidon und
b) 85 bis 50 Gew.-% Vinylacetat,
wobei sich die Gew.-% Angaben der Einzlkomponenten zu 100 % addieren.

Bevorzugt sind solche festen, oralen, pharmazeutischen und kosmetischen Zubereitungen, enthaltend als Matrix mindestens ein Copolymer mit einem K-Wert von 50 bis 200 aus
a) 20 bis 40 Gew.-% N-Vinylpyrrolidon und
b) 80 bis 60 Gew.-% Vinylacetat.

Besonders bevorzugt sind solche festen, oralen, pharmazeutischen und kosmetischen Zubereitungen, enthaltend als Matrix mindestens ein Copolymer mit einem K-Wert von 50 bis 200 aus
a) 25 bis 35 Gew.-% N-Vinylpyrrolidon und
b) 75 bis 65 Gew.-% Vinylacetat.

Die erfindungsgemäßen festen, oralen, pharmazeutischen oder kosmetischen Zubereitungen sind dadurch gekennzeichnet, daß sie das als Matrix dienende Copolymer in einer Konzentration von 0,5 bis 90 Gew.-%, bevorzugt 20 bis 80 Gew.-%, besonders bevorzugt von 30 bis 80 Gew.-% enthalten.

Unter festen, oralen, pharmazeutischen oder kosmetischen Zubereitungen sind Tabletten, Granulate, Pillen, Pastillen, Dragees, Kapseln, Pellets oder Pulver zu verstehen.

Die Zubereitungen lassen sich nach an sich bekannten Methoden u.a. durch Direkttablettierung, Trockenkompaktierung oder Feuchtgranulation sowie Feuchtextrusion der erfindungsgemäßen Copolymere mit dem pharmazeutischen oder kosmetischen Wirkstoff herstellen. Eine bevorzugtes Verfahren zur Herstellung der festen pharmazeutischen oder kosmetischen Zubereitungen ist dabei die Direkttablettierung.

Je nach Zusammensetzung der erfindungsgemäßen Copolymere lassen sich pharmazeutische oder kosmetische Zubereitungen herstellen, bei denen die Freisetzung der Wirkstoffe innerhalb einer Zeit von 0,25 bis 24 Stunden, bevorzugt 0,5 bis 20 Stunden, besonders bevorzugt innerhalb von 2 bis 16 Stunden erfolgt.

Als pharmazeutische Wirkstoffe seien hier Arzneimittel beispielsweise aus der Gruppe der Benzodiazepine, Antihypertensiva, Vitamine, Cytostatika, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Als kosmetische Wirkstoffe seien u.a. Vitamine, Riechstoffe und Parfümöle, bevorzugt Vitamine genannt.

Bei der Formulierung der Zubereitungen können selbstverständlich auch weitere, für die Herstellung von festen oralen Darreichungsformen übliche Hilfsstoffe zugesetzt werden.

Dies können u.a. sein:
Füllstoffe und Bindemittel wie z.B. Lactose, Calciumphosphate, Cellulose und Cellulosederivate, Stärke und Stärkederivate, Polyvinylpyrrolidon, Polyvinylalkohol, partiell verseiftes Polyvinylacetat, Zuckeralkohole, Zucker, Fette, Wachse;
Sprengmittel wie z.B. Kollidon® CL (Fa. BASF), Na-Carboxymethylstärke, Na-Carboxymethylcellulose;
Gleit- und Schmiermittel wie z.B. Mg-stearat, Ca-behenat, Stearinsäure, PEG;
Fließregulierungsmittel wie z.B. hochdisperses Siliciumdioxid;
Filmbildner wie z.B. Polyacrylate und Polymethacrylate (Eudragittypen), Copolymere auf Basis von Acrylatderivaten, Hydroxypropylmethylcellulose, Hydroxypropylcellulose, Celluloseacetat, Celluloseacetatphthalat und andere magensaftresistente Überzugsmaterialien;
Feuchthaltemittel wie z.B. Glycerin, Propylenglykol, Sorbitol, Mannitol, Polyethylenglykole sowie Weichmacher, Farbstoffe, Tenside, Salze, Dispergierhilfsmittel.

In den folgenden Beispielen wird die Verwendung der erfindungsgemäßen Copolymere näher erläutert.

### Beispiel 1

Jeweils 160 mg der in der Tabelle 1 aufgeführten Polymere wurden mit 160 mg Propranolol-HCl, 3,4 mg hochdispersem Siliciumdioxid und 1,6 mg Magnesiumstearat gemischt und anschließend mit einem Preßdruck von 18 KN mit 10 mm facettierten Stempeln facettiert direkttablettiert.

Die Tabletten wurden in einer Freisetzungsapparatur (Paddle-Apparatus USP XXIII) bei 37 °C und einer Umdrehungsgeschwindigkeit des Rührers von 50 rpm auf ihre Freisetzungseigenschaften geprüft. Der Wirkstoff wurde in 900 ml Phosphatpuffer pH 7,4 5 (USP XXIII) freigesetzt.

**Tabelle 1**

| Zusammensetzung | Gew.-% | K-Wert*⁾ | Freisetzung nach x min [%] | | | |
|---|---|---|---|---|---|---|
| | | | 60 | 240 | 480 | 960 |
| Vinylpyrrolidon/Vinylacetat**⁾ | 40/60 | 78,9 | 15 | 45 | 72 | 100 |
| Vinylpyrrolidon/Vinylacetat***⁾ | 40/60 | 63,3 | 15 | 34 | 55 | 90 |
| Vinylpyrrolidon/Vinylacetat | 20/80 | 63,5 | 14 | 43 | 75 | 92 |
| Vinylpyrrolidon/Vinylacetat | 30/70 | 80,0 | 16 | 36 | 51 | 78 |
| Vinylpyrrolidon/Vinylacetat | 30/70 | 60,0 | 12 | 30 | 49 | 82 |
| Vinylpyrrolidon/Vinylacetat | 40/60 | 74,4 | 19 | 53 | 85 | 100 |
| Vinylpyrrolidon/Vinylacetat | 35/65 | 70,6 | 19 | 58 | 94 | 100 |

| Vergleichsversuche: | | | | | | |
|---|---|---|---|---|---|---|
| Vinylpyrrolidon/Vinylacetat | 60/40 | 42,0 | 81 | 100 | | |
| Vinylpyrrolidon/Vinylacetat | 60/40 | 81,8 | 36 | 100 | | |
| Vinylpyrrolidon/Vinylacetat***⁾ | 60/40 | 66,1 | 31 | 100 | | |
| Vinylpyrrolidon/Vinylacetat | 30/70 | 28,9 | 84 | 93 | 100 | |

| | | | | | | |
|---|---|---|---|---|---|---|
| *⁾ Die K-Werte wurden in Ethanol bei einer Polymerkonzentration von 1 Gew.-% bestimmt. | | | | | | |
| ^{**)} vernetzte Copolymere [Vernetzer: Divinylethylenharnstoff (0,5 Gew.-%)] | | | | | | |
| ^{***)} vernetzte Copolymere [Vernetzer: Divinylethylenharnstoff (1,0 Gew.-%)] | | | | | | |

## Patentansprüche

1. Verwendung von Copolymeren mit einem K-Wert von 50 bis 200, enthaltend
a) 15 bis 50 Gew.-% N-Vinylpyrrolidon und
b) 85 bis 50 Gew.-% Vinylacetat,
wobei sich die Gew.-% Angaben der Einzelkomponenten zu 100 % addieren, als Matrix zur Herstellung von festen, oralen, pharmazeutischen und kosmetischen Darreichungsformen.

2. Verwendung von Copolymeren nach Anspruch 1, enthaltend
a) 25 bis 35 Gew.-% N-Vinylpyrrolidon und
b) 75 bis 65 Gew.-% Vinylacetat.

3. Verwendung von Copolymeren nach einem der Ansprüche 1 und 2, enthaltend 0 bis 30 Gew.-% weiterer radikalisch copolymerisierbarer Monomere c), wobei sich die Gew.-% Angaben der Einzelkomponenten a) bis c) zu 100 % addieren.

4. Verwendung von Copolymeren nach einem der Ansprüche 1 bis 3, **dadurch gekennzeichnet, daß** die Copolymeren quervernetzt sind.

5. Verwendung von Copolymeren nach einem der Ansprüche 1 bis 4, **dadurch gekennzeichnet, daß** sie durch Direkttablettierung, Trockenkompaktierung oder Feuchtgranulation sowie durch Feuchtextrusion mit dem pharmazeutischen oder kosmetischen Wirkstoff verarbeitet werden.

6. Feste, orale, pharmazeutische und kosmetische Zubereitungen, enthaltend als Matrix mindestens ein Copolymer, definiert gemäß Anspruch 1, aus
a) 15 bis 50 Gew.-% N-Vinylpyrrolidon und
b) 85 bis 50 Gew.-% Vinylacetat,
wobei sich die Gew.-% Angaben der Einzelkomponenten zu 100 % addieren.

7. Feste, orale, pharmazeutische und kosmetische Zubereitungen nach Anspruch 6, enthaltend als Matrix mindestens ein Copolymer, definiert gemäß Anspruch 2, aus
a) 25 bis 35 Gew.-% N-Vinylpyrrolidon und
b) 75 bis 65 Gew.-% Vinylacetat,
wobei sich die Gew.-% Angaben der Einzelkomponenten zu 100 % addieren.

8. Feste, orale, pharmazeutische und kosmetische Zubereitungen nach einem der Ansprüche 6 und 7, enthaltend das als Matrix dienende Copolymer in einer Konzentration von 0,5 bis 90 Gew.-%.

9. Feste, orale, pharmazeutische und kosmetische Zubereitungen nach einem der Ansprüche 6 bis 8, aus denen die Wirkstoffe innerhalb einer Zeit von 0,25 bis 24 Stunden freigesetzt werden.

## Claims

1. The use of copolymers with a K value of from 50 to 200, comprising
a) 15 to 50% by weight N-vinylpyrrolidone and
b) 85 to 50% by weight vinyl acetate,
where the % by weight data for the individual components add up to 100%, as matrix for producing solid oral pharmaceutical and cosmetic forms.

2. The use of copolymers as claimed in claim 1, comprising
a) 25 to 35% by weight N-vinylpyrrolidone and
b) 75 to 65% by weight vinyl acetate.

3. The use of copolymers as claimed in either of claims 1 and 2, comprising 0 to 30% by weight of other free-radical copolymerizable monomers c), where the % by weight data for the individual components a) to c) add up to 100%.

4. The use of copolymers as claimed in any of claims 1 to 3, wherein the copolymers are crosslinked.

5. The use of copolymers as claimed in any of claims 1 to 4, wherein they are processed by direct tableting, dry compaction or wet granulation, and by wet extrusion with the pharmaceutical or cosmetic active ingredient.

6. A solid oral pharmaceutical or cosmetic preparation comprising as matrix at least one copolymer as defined in claim 1, composed of
a) 15 to 50% by weight N-vinylpyrrolidone and
b) 85 to 50% by weight vinyl acetate,
where the % by weight data for the individual components add up to 100%.

7. A solid oral pharmaceutical or cosmetic preparation as claimed in claim 6, comprising as matrix at least one copolymer as defined in claim 2, composed of
a) 25 to 35% by weight N-vinylpyrrolidone and
b) 75 to 65% by weight vinyl acetate,
where the % by weight data for the individual components add up to 100%.

8. A solid oral pharmaceutical or cosmetic preparation as claimed in either of claims 6 and 7, comprising the copolymer serving as matrix in a concentration of from 0.5 to 90% by weight.

9. A solid oral pharmaceutical or cosmetic preparation as claimed in any of claims 6 to 8, from which the active ingredients are released within a period of from 0.25 to 24 hours.

## Revendications

1. Utilisation de copolymères ayant un indice K de 50 à 200, contenant
a) 15 à 50 % en poids de N-vinylpyrrolidone et
b) 85 à 50 % en poids d'acétate de vinyle,
tandis que la somme des indications en % en poids des composants individuels est de 100 %, comme matrice pour la préparation de formes d'administration pharmaceutiques et cosmétiques, orales, solides.

2. Utilisation de copolymères selon la revendication 1, contenant
a) 25 à 35 % en poids de N-vinylpyrrolidone et
b) 75 à 65 % en poids d'acétate de vinyle.

3. Utilisation de copolymères selon l'une des revendications 1 et 2, contenant 0 à 30 % en poids d'autres monomères c) copolymérisables par voie radicalaire, tandis que la somme des indications en % en poids des composants individuels a) à c) est de 100 %.

4. Utilisation de copolymères selon l'une des revendications 1 à 3, **caractérisée par le fait que** les copolymères sont réticulés.

5. Utilisation de copolymères selon l'une des revendications 1 à 4, **caractérisée par le fait qu'**on opère par fabrication directe de comprimés, compactage à sec ou granulation à l'état humide, ainsi que par extrusion à l'état humide, avec la substance à activité pharmaceutique ou cosmétique.

6. Compositions pharmaceutiques et cosmétiques orales, solides, contenant comme matrice au moins un copolymère défini conformément à 1 a revendication 1, résultant de
a) 15 à 50 % en poids de N-vinylpyrrolidone et
b) 85 à 50 % en poids d'acétate de vinyle,
tandis que la somme des indications en % en poids des composants individuels est de 100 %.

7. Compositions pharmaceutiques et cosmétiques orales, solides, selon la revendication 6, contenant comme matrice au moins un copolymère défini conformément à 1 a revendication 2, résultant de
a) 25 à 35 % en poids de N-vinylpyrrolidone et
b) 75 à 65 % en poids d'acétate de vinyle,
tandis que la somme des indications en % en poids des composants individuels est de 100 %.

8. Compositions pharmaceutiques et cosmétiques orales, solides, selon l'une des revendications 6 et 7, contenant le copolymère servant de matrice en une concentration de 0,5 à 90 % en poids.

9. Compositions pharmaceutiques et cosmétiques orales, solides, selon l'une des revendications 6 à 8, à partir desquelles les substances actives sont libérées sur une durée de 0,25 à 24 heures.
